(19) Europäisches Patentamt
European Patent Office
Office européen des brevets

(11) Numéro de publication: **0 114 014**
**B1**

(12) # FASCICULE DE BREVET EUROPÉEN

(45) Date de publication du fascicule du brevet: **20.05.87**

(51) Int. Cl.⁴: **C 07 D 495/04**, A 61 K 31/40 // (C07D495/04, 333:00, 209:00)

(21) Numéro de dépôt: **83402438.2**

(22) Date de dépôt: **15.12.83**

(54) **Dérivés de thiéno (2,3-b) pyrrole, leur procédé de préparation et les compositions pharmaceutiques les renfermant.**

(30) Priorité: **16.12.82 FR 8221090**

(43) Date de publication de la demande: **25.07.84 Bulletin 84/30**

(45) Mention de la délivrance du brevet: **20.05.87 Bulletin 87/21**

(84) Etats contractants désignés: **AT BE CH DE FR GB IT LI LU NL SE**

(56) Documents cité: **BSM Français 8439M**
**Medical Chemistry 3rd Edn. Part I. Page 77.**

(73) Titulaire: **ADIR, 22, rue Garnier, F-92200 Neuilly- sur- Seine (FR)**

(72) Inventeur: **Wierzbicki, Michel, 107 Rue de la République, F-92800 Puteaux (FR)**
Inventeur: **Bure, Jacques, 22 Rue Perronet, F-92200 Neuilly sur Seine (FR)**

(74) Mandataire: **Reverbori, Marcelle, ADIR 22 Rue Garnier, F-92200 Neuilly- sur- Seine (FR)**

**EP 0 114 014 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1987

**Description**

La présente invention a pour objet de nouveaux dérivés de thiéno [2,3-b] pyrrole, leur procédé de préparation et les compositions pharmaceutiques les renfermant.

Elle concerne particulièrement les dérivés de thiéno [2,3-b]pyrrole de formule générale I:

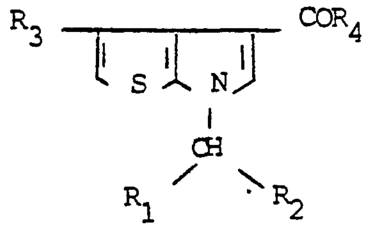

(I)

dans laquelle:

$R_1$ représente un atome d'hydrogène ou un radical alcoyle ayant de 1 à 5 atomes de carbone en chaîne droite ou ramifiée;

$R_2$ représente

un radical cyano,

un radical carboxy,

un groupe COOM dans lequel M représente un cation d'un métal alcalin ou alcalino-terreux, un radical ammonium, un radical mono-, di-, tri-, ou tétra-alcoylammonium éventuellement mono- ou poly-substitué par un radical hydroxy;

un groupe COOR dans lequel R représente un radical alcoyle ayant de 1 à 5 atomes de carbone, ou

un groupe 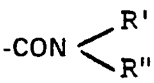

dans lequel R' et R'' identiques ou différents représentent chacun un atome d'hydrogène ou un radical alcoyle en chaîne droite ou ramifiée ayant de 1 à 5 atomes de carbone;

$R_3$ représente un radical alcoyle en chaîne droite ou ramifiée, ayant de 1 à 5 atomes de carbone, et

$R_4$ représente un radical alcoyle, en chaîne droite ou ramifiée, ayant de 1 à 5 atomes de carbone, ou un radical phényle éventuellement mono-ou-poly-substitué par un atome d'halogène, un radical alcoyle ou alcoxy ayant chacun de 1 à 5 atomes de carbone, un radical nitro ou un radical dialcoylamino dans lequel le groupe alcoyle renferme de 1 à 5 atomes de carbone, en chaîne droite ou ramifiée.

Les dérivés de formule générale I possèdent un atome de carbone asymétrique. A chacun d'eux correspond donc deux énantiomères qui sont également inclus dans la présente invention.

"L'état anterieur de la technique dans illustré notamment par le BSM français n° 8439M et par A. BURGER, Medical Chemistry Third edition, Part I page 77. Il est connu, d'après le BSM 8439M, que les 1-carboxyalcoyl 3-aroylindoles présentent des activités analgésiques et antiinflammatoires. Toutefois le remplacement dans ces indoles du cycle benzénique par un cycle thiophène conduit aux dérivés indoliques antérieurement connus une superiorite pharmacologique surprenante et inattendue dont en particulier une activité plus élevée, se manifestant plus précocement et de plus longue durée; critères particulièrement appréciables pour une meilleure utilisation thérapeutique".

La présente invention a également pour objet le procédé de préparation des dérivés de formule générale I caractérisé en ce que:

l'on dédiazote les dérivés diazoïques de formule générale II:

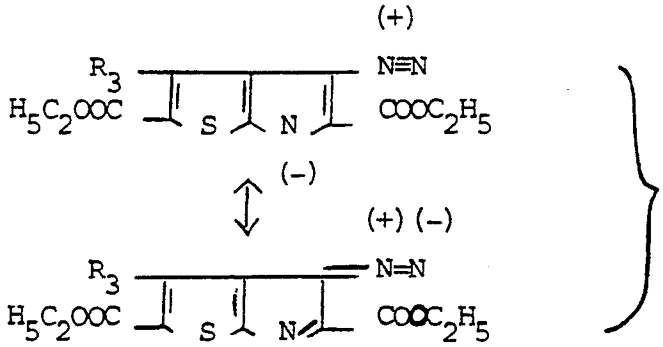

(II)

dans lesquelles $R_3$ a la signification précédemment définie pour obtenir le composé de formule générale III:

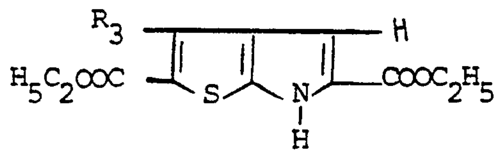

(III)

dans lequelle $R_3$ a la signification énoncée précédemment,

l'on acyle le composé III ainsi obtenu selon la méthode de Friedel-Craft au moyen d'un halogénure d'acyle de formule X-CO-$R_4$ dans laquelle X représente un atome d'halogène, notamment un atome de brome ou de chlore, et $R_4$ est tel que précédemment défini, pour obtenir le dérivé de formule générale IV:

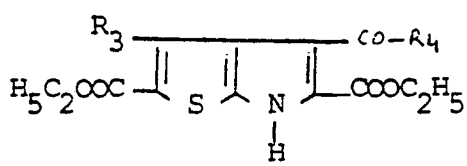

(IV)

dans laquelle $R_3$ et $R_4$ ont les significations précédemment définies,

l'on saponifie les dérivés IV pour obtenir le composés de formule générale V:

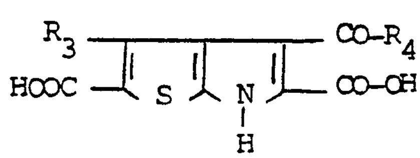

(V)

dans laquelle $R_3$ et $R_4$ ont les significations énoncées précédemment,

l'on décarboxyle ces composés V pour obtenir les dérivés de formule générale VI:

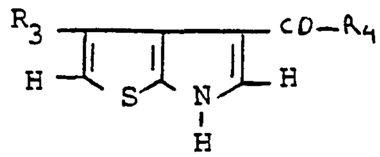

(VI)

dans laquelle $R_3$ et $R_4$ ont les significations énoncées précédement, lesquels composés VI sont, ensuite N-alcoylés un moyen d'un α-halogéno acide ou d'un dérivé de formule VII:

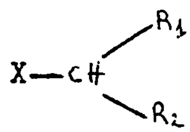

(VII)

dans laquelle $R_1$ et $R_2$ ont les significations précédemment définies et X représente un atome d'halogène tel que chlore ou brome.

Il est particulièrement avantageux d'effectuer la dédiazotation du dérivé II soit par chauffage dans un solvant adéquat tel que par exemple l'éthanol, éventuellement en catalysant la réaction par des traces d'HCl, d'U.V. ou d'initiateurs radicalaires, soit par réduction au moyen de réducteurs tels que par exemple: $LiAlH_4$, $NaBH_4$. De même, il est spécialement adéquat d'effectuer l'acylation des composés III en présence d'un catalyseur (acide de Lewis) dans un solvant approprié tel que le dichlorométhane.

La saponifications des composés IV est réalisée convenablement par chauffage des dits composés IV dans un mélange base-éthanol, suivi d'une acidification. Enfin, la décarboxylation des composés V s'effectue favorablement par chauffage dans un solvant adéquat tel que par exemple la quinoléïne en présence de poudre de cuivre comme catalyseur.

Les produits de départ de formule générale II ont été préparés selon la méthode de Wierzbicki M. et coll., Bull. Soc. Chim. France (1975) p. 1786-1792, à partir des dérivés de formule générale:

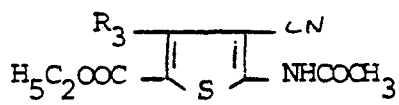

dans laquelle $R_3$ a la signification définie précédemment. Ces derniers dérivés ont eux-mêmes été préparés selon la méthode de Gewald K. et coll., Chem. Ber. (1966), 99, p.94 et 2712, à partir des matières premières de formule générale:

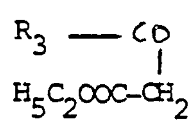

dans laquelle $R_3$ a la signification énoncée précédemment, lesquelles matières premières, traitées par $CN-CH_2-CN$, du soufre, une amine secondaire et de l'éthanol sont cyclisées en dérivés de formule générale:

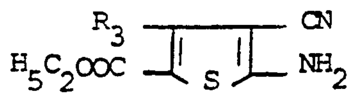

dans laquelle $R_3$ est tel que précédemment défini, dérivés qui ont été ensuite N-acétylés par $CH_3COCl/CH_3COOH$.

Les dérivés de formule générale I possèdent des propriétés pharmacologiques intéressantes, notamment principalement des propriétés analgésiques, et, à un degré moindre, des propriétés anti-inflammatoires.

Leur toxicité est faible, leur $DL_{50}$ déterminée par voie orale chez la souris se situe entre 250 et 2000 mg/kg.

Ainsi, pour les produits des exemples 7 et 9 ci-après décrits, les $DL_{50}$ per os chez la souris sont respectivement de 480 et 680 mg/kg alors que, sous les mêmes conditions elles sont respectivement de 1150 et 1400 mg/kg pour la glafénine et la clométacine, agents analgésiques bien connus pris comme produits de référence.

L'activité analgésique des dérivés de l'invention a été mise en évidence notamment par le test de Hendershot, L.C., Forsaith, J.J., J. Pharmacol. Exp. Ther. (1959), 125, 237, relatif aux crampes abdominales induites par la phénylbenzoquinone, et par le test de Koster R., Anderson M., de Beer E.S., Fed. Proc. (1959), 18, 412, relatif aux étirements à l'acide acétique. Pour chacun de ces tests, la dose efficace moyenne $DE_{50}$ se situe pour les dérivés de l'invention entre 5 et 100 mg/kg en administration orale chez la souris Swiss.

A titre indicatif, on a noté que selon le test de Hendershot ci-dessus cité, les doses efficaces moyennes $DE_{50}$, une heure après le traitement, ont été pour les composés des exemples 7 et 9 ultérieurement décrits, respectivement 21,6 et 26,7 mg/kg alors, que pour la glafénine elle était de 43 mg/kg. Par ailleurs, pour les dérivés de l'invention, la durée de l'effet analgésique est bien supérieure à celle des analgésiques de référence. Ainsi, lors du test à la phénylbenzoquinone selon Hendershot et coll. on a observé chez la souris que le traitement per os à 25 mg/kg avec le produit de l'exemple 7 ci-après décrit permet d'obtenir, 30 minutes après le traitement, un pourcentage de protection, qui atteint 60 % et se maintient durant 5 heures, alors que dans les mêmes condition avec la clométacine le pourcentage de protection n'est que de 46 % et l'activité disparait au bout de 3 heures.

L'activité anti-inflammatoire des dérivés de l'invention a été étudiée selon différents tests.

En particulier, selon le test de La Belle A. et Tislow R., J. Pharmacol. Exp. Ther. (1950), 98, 19, relatif à la douleur provoquée par une arthrite au nitrate d'argent, la dose efficace, moyenne ($DE_{50}$) des produits de l'invention se situe entre 50 et 200 mg/kg en administration orale chez le rat.

Avec le test de Sancilio L.F., J. Pharmacol. Exp. Ther. (1969), 168, 199, concernant la pleurésie à la carragénine, les produits de l'invention présentent une activité très significative entre 12,5 et 200 mg/kg en administration orale chez la souris Swiss.

Enfin, selon le test de Winter C.A., Riseley E.A., et Nuss. C.W., J. Pharmacol. Exp. Ther. (1963), 141, 369, relatif aux granulômes au coton, aucun des produits de l'invention ne donne d'activité importante à des doses inférieures à 50 mg/kg en administration orale chez le rat S.D.

Par ailleurs, avec le test de la plaque chauffante selon la méthode de Chen I.Y.P. et Beekman H., Science (1951) 113, 631, les doses actives des dérivés de l'invention, sont supérieures à 50 mg/kg en administration sous-cutanée, chez la souris NMHI, ce qui montre l'absence d'activité centrale pour les composés ainsi testés.

L'application du test de Flower R.J., Chung H.S. et Cushman D.W., Prostaglandines (1973), (4), 325, aux dérivés de l'invention a montré que certains de ces dérivés sont inhibiteurs des systèmes enzymatiques de synthèse des prostaglandines pour des doses inhibitrices moyennes ($DI_{50}$) pouvant descendre jusqu'à $10^{-6}$ M ce qui tend à prouver que les dits-produits ont un mode d'action considéré comme périphérique.

Les propriétés pharmacologiques ci-dessus décrites ainsi que la faible toxicité de dérivés de formule générale I permettent leur utilisation en thérapeutique principalement dans le traitement des douleurs aigües ou chroniques et notamment des douleurs accompagnant un processus inflammatoire (douleurs rhumatismales d'arthrose, d'arthrite, de lombo-sciatique etc...) des douleurs traumatiques, post-traumatiques ou postopératoires, des douleurs des sphères ORL, stomatologique ou génito-urinaire, et également de certaines céphalées, névralgies, migraines et douleurs cancéreuses.

La présente invention a également pour objet les compositions pharmaceutiques contenant comme principe actif: un composé de formule générale I, mélange ou associé à un excipient pharmaceutique approprié, comme par exemple, l'eau distillée, le glucose, le lactose, l'amidon, le talc, l'éthyl cellulose, le stéarate de magnésium ou le beurre de cacao.

Les compositions pharmaceutiques ainsi obtenues se présentent généralement sous forme dosée et peuvent contenir de 25 à 250 mg de principe actif. Elles peuvent revêtir par exemple, la forme de comprimés, dragées, gélules, suppositoires, solutions injectables ou buvables ou pommades, et être, selon les cas, administrées, par voie orale, rectale, parentérale ou locale à la dose de 25 à 250 mg 1 à 4 fois par jour.

Les exemples suivants illustrent l'invention, les points de fusion étant, sauf mention contraire, déterminés à la platine chauffante de Kofler.

**Exemple 1**:

Diéthoxycarbonyl-2,5 méthyl-3 thiéno[2,3-b]pyrrole:

1 mole d'acétamido-2 cyano-3 éthoxycarbonyl-5 méthyl-4 thiophène (préparé selon la méthode de Gewald K. et coll., Chem. Ber. (1966), 99, p. 94 et 2712, à partir de l'acétate d'éthyle.) est portée à reflux dans 1,6 l d'acétone avec 187 g de bromoacétate d'éthyle et 250 g de carbonate de potassium pendant 24 heures. Après quoi, le mélange est précipité sur 6 litres d'un mélange eau-glace (1/1). On agite vigoureusement, filtre le précipité obtenu, le sèche à l'air, le lave avec 500 ml d'un mélange glacé eau-éthanol (60/40), le sèche à nouveau, et le lave enfin avec 700 ml d'un mélange cyclohexanebenzène (6/1). Le produit obtenu (320 g-0,95 mole) est alors agite dans 1,2 litres d'$H_2SO_4$ à 30 %, maintenu à une temperature inferieure a -10°C, sous un courant d'azote. On additionne progressivement 85 g de nitrite de potassium dans 180 ml d'eau, en maintenant la temperature. Une fois l'addition terminée, on maintient l'agitation quelques minutes, puis on ajoute 7,2 litres d'un mélange eau-glace. On agite vigoureusement. Le précipité jaune obtenu est essoré sommairement et rincé à l'eau jusqu'à presque neutralité. Il est alors porté à reflux dans 3 litres d'éthanol jusqu'à fin du dégagement d'azote. Après quoi, on concentre le milieu réactionnel par distillation jusqu'à 1 litre, on ajoute 300 ml d'eau et agite vigoureusement. Une fois la cristallisation terminée, on filtre le précipité et le sèche. On obtient ainsi 205 g (0,73 mole) de diéthoxycarbonyl-2,5 méthyl-3 thiéno(2,3-b)pyrrole, P.F.: 139°C.

De la même manière a été préparé:

le diéthoxycarbonyl-2,5 n.propyl-3 thiéno[2,3-b] pyrrole P.F.: 137-138°C.

**Exemple 2**:

Diéthoxycarbonyl-2,5 méthyl-3 parachlorobenzoyl-4 thiéno [2,3-b]pyrrole:

70 g (0,25 mole) de diéthoxycarbonyl-2,5 méthyl-3 thiéno[2,3-b]pyrrole sont agités avec 65 g de chlorure de parachlorobenzoyle et 120 g de tétrachlorure d'étain dans 1,2 litres de dichlorométhane. L'ensemble est alors maintenu à un leger reflux pendant 20 heures. Le mélange est ensuite hydrolysé avec 500 ml d'acide chlorhydrique en solution 4N, et 500 g de glace pilée. On agite vigoureusement jusqu'à hydrolyse complète. On décante et lave la phase aqueuse avec 3 fois 200 ml de dichlorométhane. Les phases organiques réunies sont lavées à l'eau, puis avec une solution diluée de bicarbonate de sodium et enfin le solvant est évaporé. On obtient ainsi 105 g de diéthoxycarbonyl-2,5 méthyl-3 parachlorobenzoyl-4 thiéno [2,3-b] pyrrole brut qui sera utilisé ultérieurement sans autre purification.

De la même manière ont été obtenus:

a) le diéthoxycarbonyl-2,5 méthyl-3 acétyl-4 thiéno[2,3-b]pyrrole.
b) le diéthoxycarbonyl-2,5 méthyl-3 orthochloro-benzoyl-4 thiéno[2,3-b]pyrrole.
c) le diéthoxycarbonyl-2,5 méthyl-3 (dichloro-2,6 benzoyl)-4 thiéno[2,3-b]pyrrole.
d) le diéthoxycarbonyl-2,5 méthyl-3 parafluorobenzoyl-4 thiéno[2,3-b]pyrrole.
e) le diéthoxycarbonyl-2,5 méthyl-3 métachlorobenzoyl-4 thiéno[2,3-b]pyrrole.
f) le diéthoxycarbonyl-2,5 méthyl-3 benzoyl-4 thiéno[2,3-b]pyrrole.
g) le diéthoxycarbonyl-2,5 méthyl-3 paraméthoxy-benzoyl-4 thiéno[2,3-b]pyrrole.
h) le diéthoxycarbonyl-2,5 n.propyl-3 parafluoro-benzoyl-4 thiéno[2,3-b]pyrrole.
i) le diéthoxycarbonyl-2,5 n.propyl-3 orthochloro-benzoyl-4 thiéno[2,3-b]pyrrole.
j) le diéthoxycarbonyl-2,5 méthyl-3 orthométhyl-benzoyl-4 thiéno[2,3,b]pyrrole.
k) le diéthoxycarbonyl-2,5 méthyl-3 métaméthylbenzoyl-4 thiéno[2,3,b]pyrrole.
l) le diéthoxycarbonyl-2,5 méthyl-3 paraméthyl-benzoyl-4 thiéno[2,3,b]pyrrole.

**Exemple 3**:

dicarboxy-2,5 méthyl-3 parachlorobenzoyl-4 thiéno[2,3-b]pyrrole:

105 g de diéthoxycarbonyl-2,5 méthyl-3 parachloro-benzoyl-4 thiéno[2,3-b]pyrrole brut sont chauffés à reflux dans un mélange de 1 litre d'éthanol et 1 litre de solution N de soude, jusqu'à saponification totale. L'éthanol est alors éliminé par distillation au bain marie. La solution aqueuse est filtrée puis acidifiée. Le précipité obtenu est recueilli par filtration, lavé à l'eau, séché à l'air puis sous vide. On obtient ainsi 92 g de dicarboxy-2,5 méthyl-3 parachlorobenzoyl-4 thiéno[2,3-b]pyrrole brut, qui sera utilisé dans les synthèses ultérieures sans autre purification.

De la même manière ont été obtenus:

a) le dicarboxy-2,5 méthyl-3 acétyl-4 thiéno[2,3-b]pyrrole.
b) le dicarboxy-2,5 méthyl-3 orthochlorobenzoyl-4 thiéno[2,3-b]pyrrole.
c) le dicarboxy-2,5 méthyl-3 (dichloro-2,6 benzoyl)-4 thiéno[2,3-b]pyrrole.
d) le dicarboxy-2,5 méthyl-3 parafluorobenzoyl-4 thiéno[2,3-b]pyrrole
e) le dicarboxy-2,5 méthyl-3 métachlorobenzoyl-4 thiéno[2,3-b]pyrrole.
f) le dicarboxy-2,5 méthyl-3 benzoyl-4 thiéno[2,3-b]pyrrole.

g) le dicarboxy-2,5 méthyl-3 paraméthoxybenzoyl-4 thiéno[2,3-b]pyrrole.
h) le dicarboxy-2,5 n.propyl-3 parafluorobenzoyl-4 thiéno[2,3-b]pyrrole.
i) le dicarboxy-2,5 n.propyl-3 orthochlorobenzoyl-4 thiéno[2,3-b]pyrrole.
j) le dicarboxy-2,5 méthyl-3 orthométhylbenzoyl-4 thiéno[2,3,b]pyrrole.
k) le dicarboxy-2,5 méthyl-3 métaméthylbenzoyl-4 thiéno[2,3,b]pyrrole.
l) le dicarboxy-2,5 méthyl-3 paraméthylbenzoyl-4 thiéno[2,3,b]pyrrole.

**Exemple 4**:

méthyl-3 parachlorobenzoyl-4 thiéno[2,3,-b]pyrrole

92 g de dicarboxy-2,5 méthyl-3 parachlorobenzoyl-4 thiéno[2,3-b]pyrrole sont chauffés dans 900 ml de quinoléïne avec 300 mg de poudre de cuivre jusqu'à obtenir un dégagement de $CO_2$ au voisinage du reflux. Une fois ce dégagement terminé, on laisse revenir à température ambiante puis on ajoute au mélange, en refroidissant, 200 ml d'une solution 4N d'acide chlorhydrique. On agite très vigoureusement, le précipité fin obtenu est recueilli par filtration, lavé avec 2 fois 50 ml d'une solution N d'acide chlorhydrique, puis à l'eau. L'ensemble des solutions aqueuses est extrait avec 3 fois 150 ml de dichlorométhane.

La solution dans le dichlorométhane ainsi obtenue est lavée à l'eau puis évaporée à sec. Le résidu obtenu est repris par le minimum de dichlorométhane et donne un dérivé cristallisé que l'on additionne au précédent. L'ensemble est recristallisé dans l'éthanol aqueux. On obtient ainsi 56,5 g de méthyl-3 parachlorobenzoyl-4 thiéno[2,3-b]pyrrole, P.F.: 223° C. De la même manière ont été obtenus:
a) le méthyl-3 acétyl-4 thiéno]2,3-b]pyrrole, P.F.: 183° C.
b) le méthyl-3 benzoyl-4 thiéno[2,3-b]pyrrole, P.F.: 230,5° C.
c) le méthyl-3 (dichloro-2,6 benzoyl)-4 thiéno[2,3-b]pyrrole, P.F.: 219° C.
d) le n.propyl-3 orthochlorobenzoyl-4 thiéno[2,3-b]pyrrole, P.F.: 108-109° C.
e) le méthyl-3 métachlorobenzoyl-4 thiéno[2,3-b]pyrrole, P.F.: 221-222° C.
f) le méthyl-3 orthochlorobenzoyl-4 thiéno[2,3-b]pyrrole, P.F.: 186° C.
g) le méthyl-3 parafluorobenzoyl-4 thiéno[2,3-b]pyrrole, P.F.: 245° C.
h) le n.propyl-3 parafluorobenzoyl-4 thiéno[2,3-b]pyrrole, P.F.: 157° C.
i) le méthyl-3 paraméthoxybenzoyl-4 thiéno[2,3-b]pyrrole, P.F.: 215° C.
j) le méthyl-3 orthométhylbenzoyl-4 thiéno[2,3-b]pyrrole.
k) le méthyl-3 métaméthylbenzoyl-4 thiéno[2,3-b]pyrrole.
l) le méthyl-3 paraméthylbenzoyl-4 thiéno[2,3-bpyrrole.

**Exemple 5**:

Méthyl-3 parachlorobenzoyl-4 α-carboxyéthyl-6 thiéno 2,3-b pyrrole:

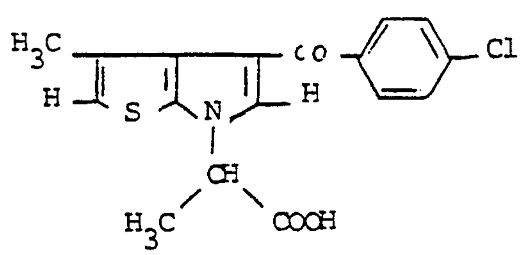

34 g de méthyl-3 parachlorobenzoyl-4 thiéno[2,3-b]pyrrole sont additionnés à une solution d'éthylate de sodium obtenue par dissolution de 5,66 g de sodium dans 400 ml d'éthanol. On ajoute alors goutte à goutte en agitant 44 g d'α-bromopropionate d'éthyle dans 150 ml d'éthanol et on maintient à reflux pendant 4 heures. Le mélange obtenu est hydrolysé sans purification par addition de 150 ml d'une solution N de soude et chauffage à reflux pendant quelques minutes.

L'éthanol est alors évaporé par distillation. Le mélange est dilué de moitié avec de l'eau, le tout est extrait par 500 ml de dichlorométhane. La phase dichlorométhane est lavée 2 fois avec 25 ml de soude normale et la phase aqueuse est lavée 2 fois avec 50 ml de dichlorométhane. Cette phase aqueuse, acidifiée à pH < 1 est à nouveau extraite au dichlorométhane. La phase organique, lavée à l'eau puis traitée au noir animal est ensuite évaporée. Le résidu cristallisé obtenu est lavé avec 200 ml d'un mélange benzène-cyclohexane 1/1. On obtient ainsi 36 g de méthyl-3 parachlorobenzoyl-4 α-carboxyéthyl-6 thiéno[2,3-b]pyrrole, P.F.: 149° C.

**Exemple 6 à 16**:

Les dérivés suivants ont été préparés selon le procédé décrit dans l'exemple 5.
6) méthyl-3 parachlorobenzoyl-4 carboxyméthyl-6 thiéno[2,3-b]pyrrole, P.F.: 199° C.
7) méthyl-3 orthochlorobenzoyl-4 α-carboxyéthyl-6 thiéno[2,3-b]pyrrole, P.F.: 188° C.
8) méthyl-3 (dichloro-2,6 -benzoyl)-4 α-carboxy-éthyl-6 thiéno[2,3-b]pyrrole, P.F.: 186° C.
9) méthyl-3 benzoyl-4 α-carboxyéthyl-6 thiéno[2,3-b]pyrrole, P.F.: 152° C.
10) n.propyl-3 parafluorobenzoyl-4 carboxyméthyl-6 thiéno[2,3-b]pyrrole, P.F.: 199° C.
11) méthyl-3 orthochlorobenzoyl-4 carboxyméthyl-6 thiéno[2,3-b]pyrrole, P.F.: 188° C.
12) n.propyl-3 orthochlorobenzoyl-4 α-carboxyéthyl-6 thiéno[2,3-b]pyrrole, P.F.: 158° C.
13) méthyl-3 acétyl-4 carboxy méthyl-6 thiéno[2,3-b]pyrrole, P.F.: 190° C.
14) méthyl-3 parafluorobenzoyl-4 α-carboxyéthyl-6 thiéno[2,3-b]pyrrole.
15) méthyl-3 métachlorobenzoyl-4 α-carboxyéthyl-6 thiéno[2,3-b]pyrrole.
16) méthyl-3 paraméthoxybenzoyl-4'carboxyméthyl-6 thiéno[2,3-b]pyrrole.
17) méthyl-3 orthométhylbenzoyl-4 α-carboxyéthyl-6 thiéno[2,3-b]pyrrole, P.F.: 142° C.
18) méthyl-3 métaméthylbenzoyl-4 α-carboxyéthyl-6 thiéno[2,3-b]pyrrole, P.F.: 149° C.
19) méthyl-3 paraméthylbenzoyl-4 α-carboxyéthyl-6 thiéno[2,3-b]pyrrole.

Les composés des exemples 14 à 16 sont mal cristallisés ou huileux. On a préparé leur sel de sodium par dissolution de l'acide dans une quantité stoechiométrique de soude $\frac{N}{10}$, évaporation de l'eau et séchage du résidu obtenu.

Les produits ainsi obtenus sont des solides amorphes et hygroscopiques, qui ont été testés sous cette forme.

**Exemples 20 à 24**

Les sels des dérivés acides de formule I ont été préparés comme suit:

- Le dérivé acide, en solution dans un solvant adéquat, tel que par exemple l'éthanol, le chlorure de méthylène ou le benzène, est neutralisé par une quantité stoechiométrique de base telle que par exemple la soude, les carbonates ou bicarbonates, les amines, alcoylamines et hydroxy alcoylamines. Le produit obtenu par évaporation du solvant est recristallisé dans un solvant ou un mélange de solvants adéquats tels que par exemple l'éthanol, l'éther éthylique, le benzène ou le cyclohexane.

Selon cette méthode ont été préparés les produits objet des exemples suivants:
20) sel de sodium du benzoyl-4 α-carboxyéthyl-6 méthyl-3 thiéno[2,3-b]pyrrole.
21) sel de tertiobutylamine du benzoyl-4 α-carboxy éthyl-6 méthyl-3 thiéno[2,3-b]pyrrole, P.F.: 149° C.
22) sel de diméthylaminoéthanol du benzoyl-4 α-carboxyéthyl-6 méthyl-3 thiéno[2,3-b]pyrrole, P.F.: 112° C.
23) sel de choline du benzoyl-4 α-carboxyéthyl-6 méthyl-3 thiéno[2,3-b]pyrrole, P.F.: 155° C.
24) sel de diméthylaminoéthanol de l'α-carboxyéthyl-6 orthochlorobenzoyl-4 méthyl-3 thiéno[2,3-b]pyrrole, P.F.: 101° C.

**Revendications**

pour les états contractants BE, CH, DE, FR, GB, IT, LI, LU, NL, SE

1) Les dérivés de thiéno[2,3-b]pyrrole et leurs énantiomères, se formule générale I:

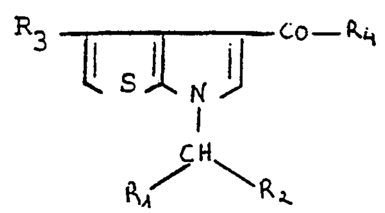

(I)

dans laquelle:

$R_1$ représente un atome d'hydrogène ou un radical alcoyle ayant de 1 à 5 atomes de carbone, en chaîne droite ou ramifiée;

$R_2$ représente
un radical cyano,
un radical carboxy,

un groupe -COOM dans lequel M représente un cation d'un métal alcalin ou alcalino-terreux, un radical ammonium, un radical mono-, di-, tri-, ou tétra-alcoylammonium éventuellement mono- ou poly-substitué par un radical hydroxy;

un groupe COOR dans lequel R représente un radical alcoyle ayant de 1 à 5 atomes de carbone,

ou un groupe -CON $<^{R'}_{R''}$

dans lequel R' et R'' identiques ou différents représentent chacun un atome d'hydrogène ou un radical alcoyle en chaîne droite ou ramifiée ayant de 1 à 5 atomes de carbone;

$R_3$ représente un radical alcoyle en chaîne droite ou ramifiée, ayant de 1 à 5 atomes de carbone, et

$R_4$ présente un radical alcoyle en chaîne droite ou ramifiée, ayant de 1 à 5 atomes de carbone, ou un radical phényle éventuellement mono- ou poly-substitué par un atome d'halogène, un radical alcoyle ou alcoxy ayant chacun de 1 à 5 atomes de carbone, un radical nitro ou un radical dialcoylamino dans lequel le groupe alcoyle renferme de 1 à 5 atomes de carbone, en chaîne droite ou ramifiée.

2) le méthyl-3 parachlorobenzoyl-4 α-carboxyéthyl-6 thiéno[2,3-b]pyrrole.

3) le méthyl-3 parachlorobenzoyl-4 carboxyméthyl-6 thiéno[-2,3-b]pyrrole.

4) le méthyl-3 orthochlorobenzoyl-4 α-carboxyéthyl-6 thiéno[2,3-b]pyrrole.

5) le méthyl-3 (dichloro-2,6 benzoyl)-4 α-carboxy-éthyl-6 thiéno[2,3-b]pyrrole.

6) le méthyl-3 benzoyl-4 α-carboxyéthyl-6 thiéno[2,3-b]pyrrole.

7) le n.propyl-3 parafluorobenzoyl-4 carboxyméthyl-6 thiéno[2,3-b]pyrrole.

8) le méthyl-3 orthochlorobenzoyl-4 carboxyméthyl-6 thiéno[2,3-b]pyrrole.

9) le n.propyl-3 orthochlorobenzoyl-4 α-carboxyéthyl-6 thiéno[2,3-b]pyrrole.

10) le méthyl-3 acétyl-4 carboxyméthyl-6 thiéno[-2,3-b]pyrrole.

11) le procédé de préparation des composés de la revendication 1, caractérisé en ce que:

l'on dédiazote les dérivés diazoïques de formule générale II:

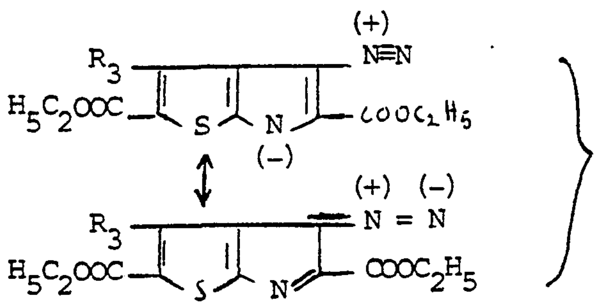

(II)

dans lesquelles $R_3$ a la signification définie dans la revendications 1, pour obtenir le composé de formule générale III:

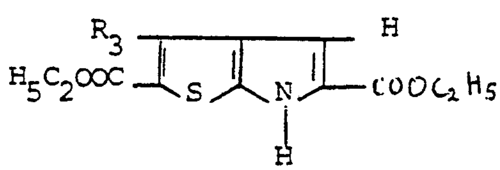

(III)

dans laquelle $R_3$ a la signification énoncée dans la revendication 1,

l'on acyle le composé III ainsi obtenu selon la méthode de Friedel-Craft au moyen d'un halogénure d'acyle de formule $X-CO-R_4$ dans laquelle $R_4$ a la signification définie dans la revendication 1, et X représente un atome d'halogène, pour obtenir le dérivé de formule générale IV:

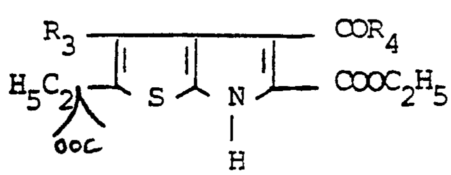

(IV)

dans laquelle $R_3$ et $R_4$ ont les significations énoncées dans la revendication 1, l'on saponifie les dérivés IV pour obtenir les composés de formule générale V:

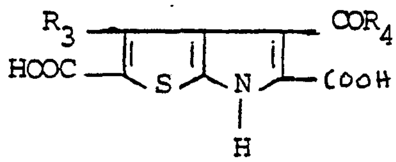

(V)

dans laquelle $R_3$ et $R_4$ ont les significations définies dans la revendication 1, l'on décarboxyle ces composés V pour obtenir les dérivés de formule générale VI:

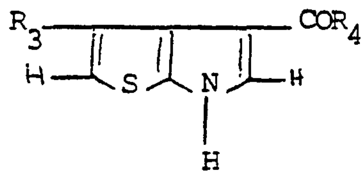

(VI)

dans laquelle $R_3$ et $R_4$ ont les significations définies dans la revendication 1, lesquels composés VI sont ensuite N-alcoylés au moyen d'un α-halogéno acide ou d'un dérivé de formule VII:

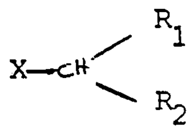

(VII)

dans laquelle $R_1$ et $R_2$ ont les significations définies dans la revendication 1, et X représente un atome d'halogène.

12) Le procédé de préparation selon la revendication 11 caractérisé en ce que la dédiazotation des dérivés II est effectuée par chauffage dans un solvant approprié en catalysant éventuellement la réaction par des traces d'HCl,-d'U.V. ou d'initiateurs radicalaires, ou par action d'un réducteur.

13) Le procédé selon les revendications 11 et 12 caractérisé en ce que l'acylation des composés III est effectuée en présence d'un catalyseur, dans un solvant approprié.

14) Le procedé selon les revendications 11 à 13 caractérisé en ce que la saponification des composés IV est réalisée par chauffage des composés IV dans un mélange base-ethanol, suivi d'une acidification.

15) Le procédé selon les revendications 11 à 14 caractérisé en ce que la décarboxylation des composés V s'effectue par chauffage dans un solvant adéquat, en présence de poudre de cuivre comme catalyseur.

16) Les compositions pharmaceutiques contenant comme principe actif, un composé selon les revendications 1 à 10, avec des excipients pharmaceutiques appropries.

17) Les compositions pharmaceutiques selon la revendication 16 présentées sous une forme convenant notamment pour le traitement des douleurs aigües ou chroniques.

**Revendications**

pour l'état contractant AT

1) Procédé de préparation des dérivés de thiéno[2,3-b]pyrrole et de leurs énantiomères, de formule générale I

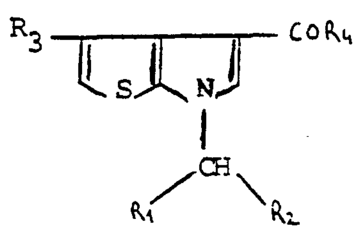

(I)

dans laquelle:

$R_1$ représente un atome d'hydrogène ou un radical alcoyle ayant de 1 à 5 atomes de carbone, en chaîne droite ou ramifiée;

$R_2$ représente

un radical cyano,

un radical carboxy,

un groupe -COOM dans lequel M représente un cation d'un métal alcalin ou alcalino-terreux, un radical ammonium, un radical mono-, di-, tri-, ou tétra-alcoylammonium éventuellement mono- ou poly-substitué par un radical hydroxy;

un groupe COOR dans lequel R représente un radical alcoyle ayant de 1 à 5 atomes de carbone,

ou un groupe $-CON{<}^{R'}_{R''}$

dans lequel R' et R'' identiques ou différents représentent chacun un atome d'hydrogène ou un radical alcoyle en chaîne droite ou ramifiée ayant de 1 à 5 atomes de carbone;

$R_3$ représente un radical alcoyle en chaîne droite ou ramifiée, ayant de 1 à 5 atomes de carbone, et

$R_4$ représente un radical alcoyle en chaîne droite ou ramifiée, ayant de 1 à 5 atomes de carbone, ou un radical phényle éventuellement mono- ou poly-substitue par un atome d'halogène, un radical alcoyle ou alcoxy ayant chacun de 1 à 5 atomes de carbone, un radical nitro ou un radical dialcoylamino dans lequel le groupe alcoyle renferme de 1 à 5 atomes de carbone, en chaine droite ou ramifiée,

caractérisé en ce que:

- l'on dédiazote les dérivés diazoïques de formule générale II:

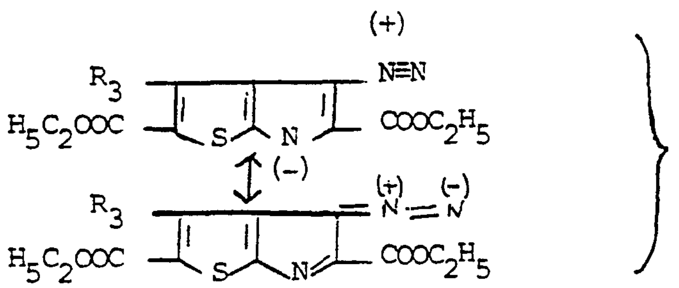

(II)

dans lesquelles $R_3$ a la signification definie ci-dessus pour obtenir le composé de formule générale III

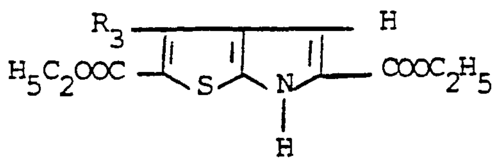

(III)

dans laquelle $R_3$ a la signification énoncée précédemment,

- l'on acyle le composé III ainsi obtenu selon la méthode de Friedel-Craft au moyen d'un halogénure d'acyle de formule X-CO-$R_4$ dans laquelle $R_4$ a la signification définie précédemment et X représente un atome d'halogène, pour obtenir le dérivé de formule générale IV:

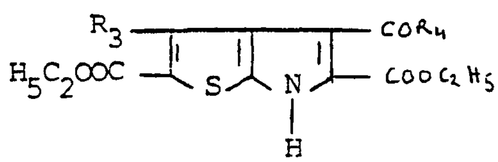

(IV)

dans laquelle $R_3$ et $R_4$ ont les significations énoncées précédemment,

- l'on saponifie les dérivés IV pour obtenir les composés de formule générale V:

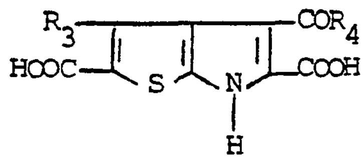

(V)

dans laquelle $R_3$ et $R_4$ ont les significations précédemment définies,

- l'on décarboxyle ces composés V pour obtenir les dérivés de formule générale VI:

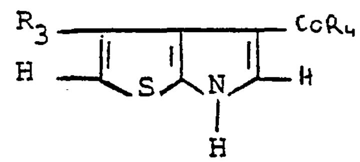

(VI)

dans laquelle $R_3$ et $R_4$ ont les significations définies précédemment,

- lesquels composés VI sont ensuite N-alcoylés au moyen d'un α-halogeno acide ou d'un dérivé de formule VII:

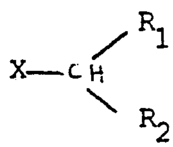

(VII)

dans laquelle $R_1$ et $R_2$ ont les significations précédemment définies, et X représente un atome d'halogène.

2) Le procédé de préparation selon la revendication 1 caractérisé en ce que la dédiazotation des dérivés II est effectué par chauffage dans un solvant approprié en catalysant éventuellemnt la réaction par des traces d'HCl, d'U.V. ou d'initiateurs radicalaires, ou par action d'un réducteur.

3) Procédé selon les revendications 1 et 2 caractérisé en ce que l'acylation des composés III est effectuée en présence d'un catalyseur, dans un solvant approprié.

4) Procédé selon les revendications 1 et 3 caractérisé en ce que la saponification des composés IV est réalisée par chauffage des composés IV dans un mélange base-éthanol, suivi d'une acidification.

5) Procédé selon les revendications 1 à 4 caractérisé en ce que la décarboxylation des composés V s'effectue par chauffage dans un solvant adequat, en présence de poudre de cuivre comme catalyseur.

**Patentansprüche**

für die Vertragsstaaten BE, CH, DE, FR, GB, IT, LI, LU, NL, SE

1. Derivate des Thieno[2,3-b]pyrrols und seine Enantiomeren der allgemeinen Formel 1

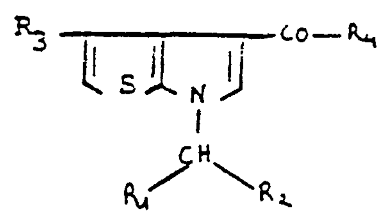

(I)

worin $R_1$ ein Wasserstoffatom oder eine geradkettige oder verzweigte Alkylgruppe mit 1 bis 5 Kohlenstoffatomen,

$R_2$ eine Cyanogruppe, eine Carboxygruppe, eine -COOM - Gruppe, in der M ein Alkali- oder Erdalkalimetallkation, eine Ammonium Di-, Tri- oder Tetraalkylammoniumgruppe, die einfach oder mehrfach durch eine Hydroxygruppe substituiert sein kann darstellt,

eine COOR-Gruppe, in der R eine Alkylgruppe mit 1 bis 5 Kohlenstoffatomen darstellt oder eine Gruppe der Formel

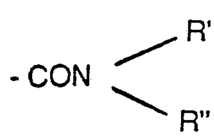

in der R' und R'', die gleich oder voneinander verschieden sind, jeweils Wasserstoffatome oder geradkettige oder verzweigte Alkylgruppen mit 1 bis 5 Kohlenstoffatomen darstellen,

$R_3$ eine geradkettige oder verzweigte Alkylgruppe mit 1 bis 6 Kohlenstoffatomen und

$R_4$ eine geradkettige oder verzweigte Alkylgruppe mit 1 bis 5 Kohlenstoffatomen oder eine Phenylgruppe, die gegebenenfalls einfach oder mehrfach durch ein Halogenatom, eine Alkyl oder Alkoxygruppe mit 1 bis 6 Kohlenstoffatomen, eine Nitrogruppe oder eine Dialkylaminogruppe, in der die geradkettige oder verzweigte Alkylgruppe 1 bis 5 Kohlenstoffatome enthält substituiert sein kann, bedeuten.

2. 3-Methyl-4-parachlorbenzoyl-6α-carboxyethyl-thieno[2,3-b]pyrrol

3. 3-Methyl-4-parachiorbenzoyi-6-carboxymethyi-thieno[ 2,3-b]pyrrol.

4. 3-Methyl-4- orthochlorobenzoyi-6α-carboxyethyl-thieno[2,3-b]pyrrol.

5. 3-Methyl-4-(2,6-dichlorbenzoyl)-6α-carboxyethyl-thieno[2,3-b]pyrrol.

6. 3-Methyl-4-benzoyl-6α-carboxethyl-thieno[2,3-b]pyrrol.

7. 3n-Propyl-4-parafluorbenzoyi-6-carboxyrnethyi-thieno[2,3-b]pyrrol.

8. 3-Methyl-4-orthochlorbenzoyl-6-carboxymethyl-thieno[2,3-b]pyrrol.

9. 3n-Propyl-4-orthochiorbenzoyl-6α-carboxyethyl-thieno[2,3-b]pyrrol.

10. 3-Methyl-4-acetyl-6-carboxymethyl-thieno[2,3-b]pyrrol.

11. Verfahren zur Herstellung der Verbindungen nach Anspruch 1, dadurch gekennzeichnet, daß man die Diazoderivate der allgemeinen Formel II

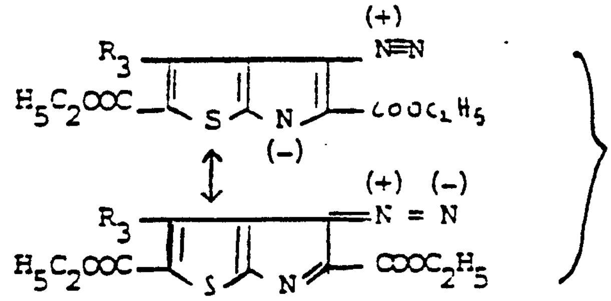

(II)

worin $R_3$ die gleichen Bedeutungen wie in Anspruch 1 hat, unter Bildung einer Verbindung der allgemeinen Formel III

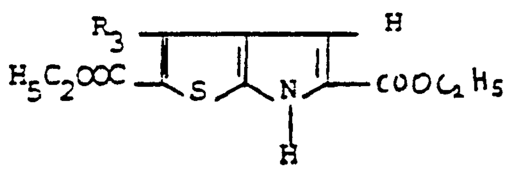

(III)

worin $R_3$ die gleichen Bedeutungen wiein Anspruch 1 hat, entdiazotiert,

die erhaltene Verbindung (III) nach der Methode von Friedel-Crafts mittels eines Acylhalogenids der Formel $X\text{-}CO\text{-}R_4$,in der $R_4$ die gleichen Bedeutungen wie in Anspruch 1 hat und X ein Halogenatom darstellt, unter Bildung eines Derivate der allgemeinen Formel IV

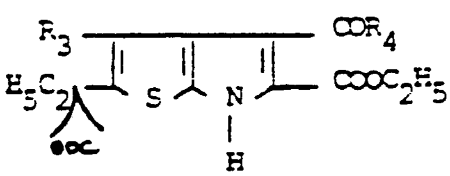

(IV)

worin $R_3$ und $R_4$ die gleichen Bedeutungen wie in Anspruch 1 haben, acyliert, die Derivate IV unter Bildung der Verbindungen der allgemeinen Formel V

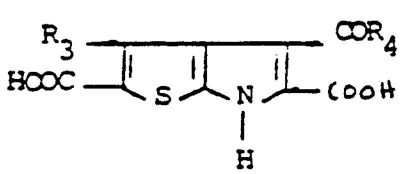

(V)

worin $R_3$ und $R_4$ die gleichen Bedeutungen wie in Anspruch 1 haben, verseift, die Verbindungen V unter Bildung von Derivaten der allgemeinen Formel VI

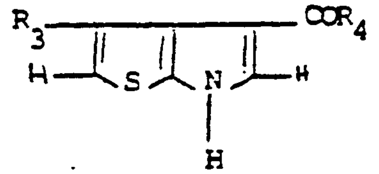

(VI)

worin $R_3$ und $R_4$ die gleichen Bedeutungen wie in Anspruch 1 haben, decarboxyliert,
und
diese Verbindungen VI dann mittels einer α-Halogensäure oder eines Derivats der Formel VII

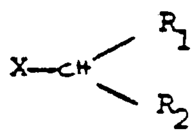

(VII)

worin $R_1$ und $R_2$ gleichen Bedeutungen wie in Anspruch 1 haben und X ein Halogenatom bedeutet, N-alkyliert

12. Verfahren nach Anspruch 11, dadurch gekennzeichnet, daß man die Entdiazotierung der Derivate II durch Erhitzen in einem geeigneten Lösungsmittel durchführt, wobei man die Umsetzung gegebenenfalls durch Spuren von HCl, UV oder Radikalinitiatoren oder Einfluß eines Reduktionsmittels katalysiert.

13. Verfahren nach den Ansprüchen 11 und 12, dadurch gekennzeichnet, daß man die Acylierung der Verbindungen III in Gegenwart eines Katalysators und in einem geeigneten Lösungsmittel durchführt.

14. Verfahren nach den Ansprüchen 11 bis 13, dadurch gekennzeichnet, daß man das Versehen der Verbindungen IV in der Weise durchführt, daß man die Verbindung IV in einer Basen/Ethanol-Mischung erhitzt und anschließend ansäuert.

15. Verfahren nach den Ansprüchen 11 bis 14, dadurch gekennzeichnet, daß man die Decarboxylierung der Verbindungen V durch Erhitzen in einem entsprechenden Lösungsmittel in Gegenwart von Kupferpulver als Katalysator durchführt.

16. Pharmazeutische Zusammensetzungen, enthaltend als aktiven Bestandteil eine Verbindung nach den Ansprüchen 1 bis 10 und geeignete pharmazeutische Hilfsstoffe.

17. Pharmazeutische Zusammensetzungen nach Anspruch 16, in einer Form, die insbesondere für die Behandlung von akuten oder chronischen Schmerzen geeignet ist.

**Patentansprüche**

für den Vertragsstaat AT

1. Verfahren zur Herstellung von Derivaten des Thieno[2,3-b]pyrrols und ihren Enantiomeren der allgemeinen Formel I

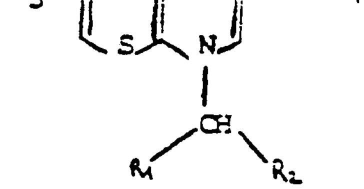

(I)

worin $R_1$ ein Wasserstoffatom oder eine geradkettige oder verzweigte Alkylgruppe mit 1 bis 5 Kohlenstoffatomen,
$R_2$ eine Cyanogruppe, eine Carboxygruppe, eine -COOM - Gruppe, in der M ein Alkali- oder

Erdalkalimetallkation,eine Ammoniumgruppe, eine Mono-, Di-, Tri- oder Tetraalkylammoniumgruppe, die einfach oder mehtfach durch eine Hydroxygruppe substituiert sein kann, darstellt,

eine COOR-Gruppe, in der R eine Alkylgruppe mit 1 bis 5 Kohlenstoffatomen darstellt oder eine Gruppe der Formel

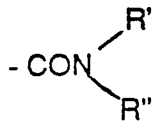

in der R' und R'', gleichartig oder voneinander verschieden sind, jeweils Wasserstoffatome oder geradkettige oder verzweigte Alkylgruppen mit 1 bis 5 Kohlenstoffatomen darstellen,

$R_3$ eine geradkettige oder verzweigte Alkylgruppe mit 1 bis 5 Kohlenstoffatomen und

$R_4$ eine geradkettige oder verzweigte Alkylgruppe mit 1 bis 5 Kohlenstoffatomen oder eine Phenylgruppe, die gegebenenfalls einfach oder mehrfach durch ein Halogenatom eine Alkyl- oder Alkoxygruppe mit 1 bis 5 Kohlenstoffatomen, eine Nitrogruppe oder eine Dialkylaminogruppe, in der die geradkettige oder verzweigte Alkylgruppe 1 bis 5 Kohlenstoffatome enthält, substituiert sein kann, bedeuten, dadurch gekennzeichnet, daß man die Diazoderivate der allgemeinen Formel II

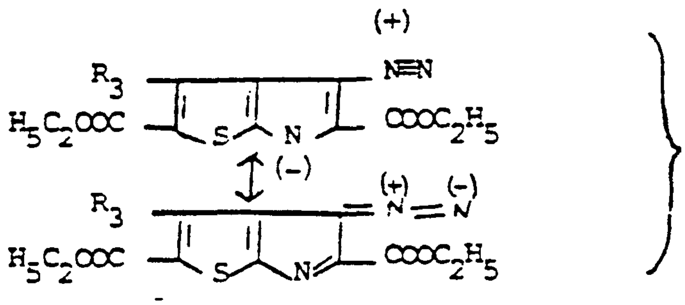

(II)

worin $R_3$ die oben angegebenen Bedeutungen hat, unter Bildung einer Verbindung der allgemeinen Formel III

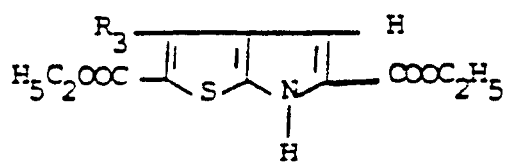

(III)

worin $R_3$ die oben agegebenen Bedeutungen hat, entdiazotiert,

die erhaltene Verbindung (III) nach der Methode von Friedel-Crafts mittels eines Acylhalogenids der Forrnel X-CO-$R_4$, in der $R_4$ die oben angegebenen Bedeutungen hat und X ein Halogenatom darstellt, unter Bildung eines Derivats der allgemeinen Formel IV

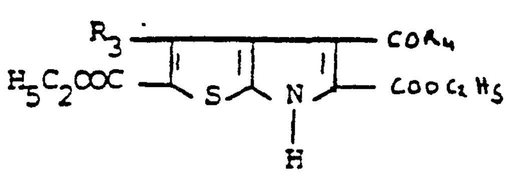

(IV)

worin $R_3$ und $R_4$ die oben angegebenen Bedeutungen haben, acyliert, die Derivate IV unter Bildung der Verbindungen der allgemeinen Formel V

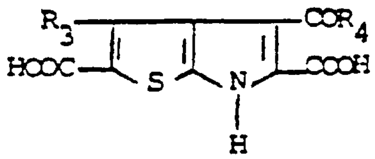

(V)

worin $R_3$ und $R_4$ die oben angegebenen Bedeutungen haben, verseift, die Verbindungen V unter Bildung von Derivaten der allgemeinen Formel VI

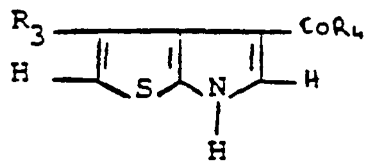

(VI)

worin $R_3$ und $R_4$ die oben angegebenen Bedeutungen haben, decarboxyliert, und diese Verbindungen VI. dann mittels einer α-Halogensäure oder eines Derivates der Formel VII

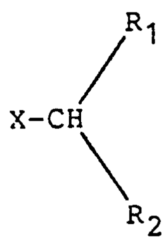

(VII)

worin $R_1$ und $R_2$ die oben angegebenen Bedeutungen haben und X ein Halogenatom bedeutet, N-alkyliert.

2. Herstellungsverfahren nach Anspruch 1, dadurch gekennzeichnet, daß man die Entdiazotierung des Derivates II durch Erhitzten in einem geeigneten Lösungsmittel durchführt, wobei man die Umsetzung gegebenenfalls durch Spuren von HCl, UV oder Radikalinitiatoren oder Einfluß eines Reduktionsmittels katalysiert.

3. Verfahren nach den Ansprüchen 1 und 2, dadurch gekennzeichnet, daß man die Acylierung der Verbindung III in Gegenwart eines Katalysators und in einem geeigneten Lösungsmittel durchführt.

4. Verfahren nach den Ansprüchen 1 bis 3, dadurch gekennzeichnet, daß man das Verseifen der Verbindung IV in der Weise durchführt, daß die Verbindung IV in einer Basen/Ethanol-Mischung erhitzt und anschließend ansäuert wird.

5. Verfahren nach den Ansprüchen 1 bis 4, dadurch gekennzeichnet, daß man die Decarboxylierung der Verbindung V durch Erhitzen in einem entsprechenden Lösungsmittel in Gegenwart von Kupferpulver als Katalysator durchführt.

## Claims

for the contracting states BE, CH, DE, FR, GB, IT, LI, LU, NL, SE

1. Derivatives of thieno[2,3-b]pyrrole and the enantiomers thereof, of the general formula I:

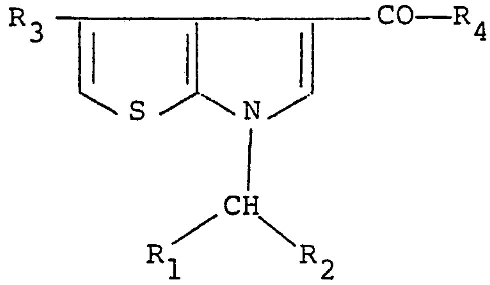

(I)

in which

$R_1$ represents a hydrogen atom or a straight- or branched-chain alkyl radical having from 1 to 5 carbon atoms,

$R_2$ represents

a cyano radical,

a carboxy radical,

a -COOM group in which M represents an alkali metal or alkaline earth metal cation, an ammonium radical or a mono-, di-, tri- or tetra-alkylammonium radical which is optionally mono- or poly-substituted by a hydroxy radical, a -COOR group in which R represents an alkyl radical having from 1 to 5 carbon atoms, or a

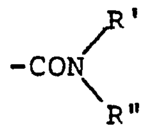

group in which R' and R'', which may be the same or different, each represents a hydrogen atom or a straight- or branched-chain alkyl radical having from 1 to 5 carbon atoms,

$R_3$ represents a straight- or branched-chain alkyl radical having from 1 to 5 carbon atoms, and

$R_4$ represents a straight- or branched-chain alkyl radical having from 1 to 5 carbon atoms, or a phenyl radical which is optionally mono- or polysubstituted by a halogen atom, an alkyl or alkoxy radical each having from 1 to 5 carbon atoms, a nitro radical or a dialkylamino radical in which the alkyl group contains from 1 to 5 carbon atoms in a straight or branched chain.

2. 3-methyl-4-para-chlorobenzoyl-6-(α-carboxyethyl)-thieno[2,3-b]pyrrole.

3. 3-methyl-4-para-chlorobenzoyl-6-carboxymethyl-thieno[2,3-b]pyrrole.

4. 3-methyl-4-ortho-chlorobenzoyl-6-(α-carboxyethyl)-thieno[2,3-b]pyrrole.

5. 3-methyl-4-(2,6-dichlorobenzoyl)-6-(α-carboxy-ethyl)-thieno[2,3-b]pyrrole.

6. 3-methyl-4-benzoyl-6-(α-carboxyethyl)-thieno-[2,3-b]pyrrole.

7. 3-n-propyl-4-para-fluorobenzoyl-6-carboxymethyl-thieno[2,3-b]pyrrole.

8. 3-methyl-4-ortho-chlorobenzoyl-6-carboxymethyl-thieno[2,3-b]pyrrole.

9. 3-n-propyl-4-ortho-chlorobenzoyl-6-(α-carboxy-ethyl)-thieno[2,3-b]pyrrole.

10. 3-methyl-4-acetyl-6-carboxymethylthieno[2,3-b]-pyrrole.

11. Process for the preparation of the compounds of claim 1, characterised in that the diazo derivatives of the general formula II:

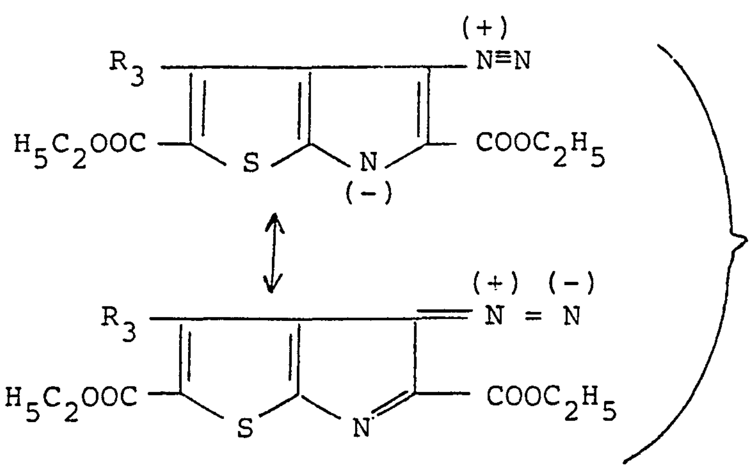

(II)

in which $R_3$ has the meaning defined in claim 1, are de-diazotised to obtain the compound of the general formula III:

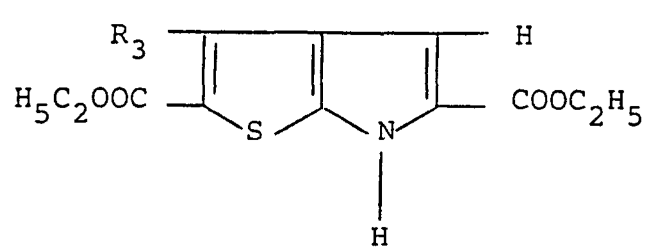

(III)

in which $R_3$ has the meaning given in claim 1,

the compound III so obtained is acylated according to the Friedel-Crafts method by means of an acyl halide of the formula X-CO-$R_4$ in which $R_4$ has the meaning defined in claim 1 and X represents a halogen atom, to obtain the derivative of the general formula IV:

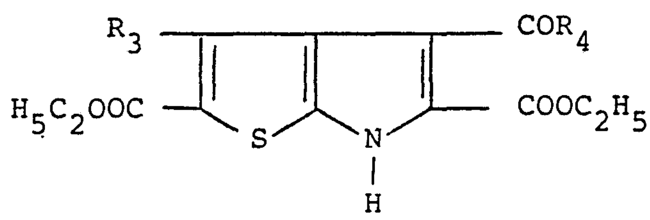

(IV)

in which $R_3$ and $R_4$ have the meanings given in claim 1,

the derivatives IV are hydrolysed to obtain the compounds of the general formula V:

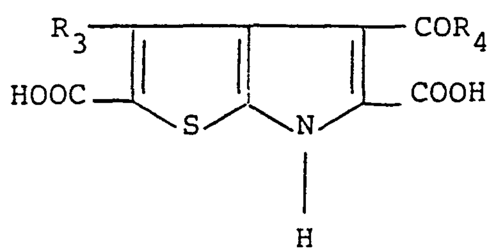

(V)

in which $R_3$ and $R_4$ have the meanings defined in claim 1,

these compounds V are decarboxylated to obtain the derivatives of the general formula VI:

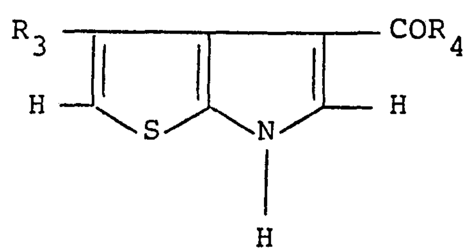

(VI)

in which $R_3$ and $R_4$ have the meanings defined in claim 1,

which compounds VI are then N-alkylated by means of an α-halo acid or a derivative of the formula VII:

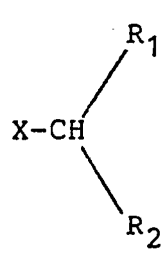

(VII)

in which $R_1$ and $R_2$ have the meanings defined in claim 1 and X represents a halogen atom.

12. Process of preparation according to claim 11, characterised in that the de-diazotisation of the derivatives II is carried out by heating in an appropriate solvent, optionally with catalysis of the reaction by traces of HCl, U.V. or radical initiators, or by the action of a reducing agent.

13. Process according to claims 11 and 12, characterised in that the acylation of the compounds III is carried out in the presence of a catalyst in an appropriate solvent.

14. Process according to claims 11 to 13, characterised in that the hydrolysis of the compounds IV is carried out by heating the compounds IV in a baseethanol mixture, followed by acidification.

15. Process according to claims 11 to 14, characterised in that the decarboxylation of the compounds V is carried out by heating in a suitable solvent in the presence of copper powder as catalyst.

16. Pharmaceutical compositions containing as active ingredient a compound according to claims 1 to 10 together with appropriate pharmaceutical excipients.

17. Pharmaceutical compositions according to claim 16 in a form suitable especially for the treatment of acute or chronic pain.

**Claims**

for the contracting state AT

1. Process for the preparation of derivatives of thieno[2,3-b]pyrrole and the enantiomers thereof, of the general formula I:

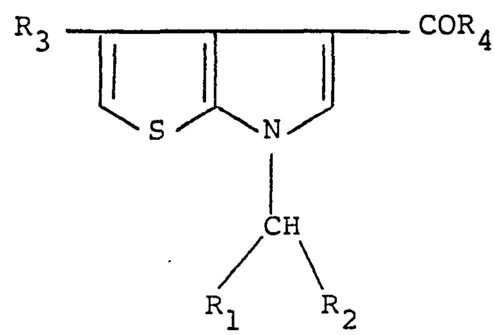

(I)

in which

$R_1$ represents a hydrogen atom or a straight- or branched-chain alkyl radical having from 1 to 5 carbon atoms,

$R_2$ represents

a cyano radical,

a carboxy radical,

a -COOM group in which M represents an alkali metal or alkaline earth metal cation, an ammonium radical or a mono-, di-, tri- or tetra-alkylammonium radical which is optionally mono- or polysubstituted by a hydroxy radical, a -COOR group in which R represents an alkyl radical having from 1 to 5 carbon atoms, or a

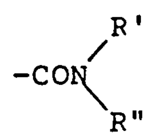

group in which R′ and R″, which may be the same or different, each represents a hydrogen atom or a straight- or branched-chain alkyl radical having from 1 to 5 carbon atoms,

$R_3$ represents a straight- or branched-chain alkyl radical having from 1 to 5 carbon atoms, and

$R_4$ represents a straight- or branched-chain alkyl radical having from 1 to 5 carbon atoms, or a phenyl radical which is optionally mono- or polysubstituted by a halogen atom, an alkyl or alkoxy radical each having from 1 to 5 carbon atoms, a nitro radical or a dialkylamino radical in which the alkyl group contains from 1 to 5 carbon atoms in a straight or branched chain,

characterised in that

the diazo derivatives of the general formula II:

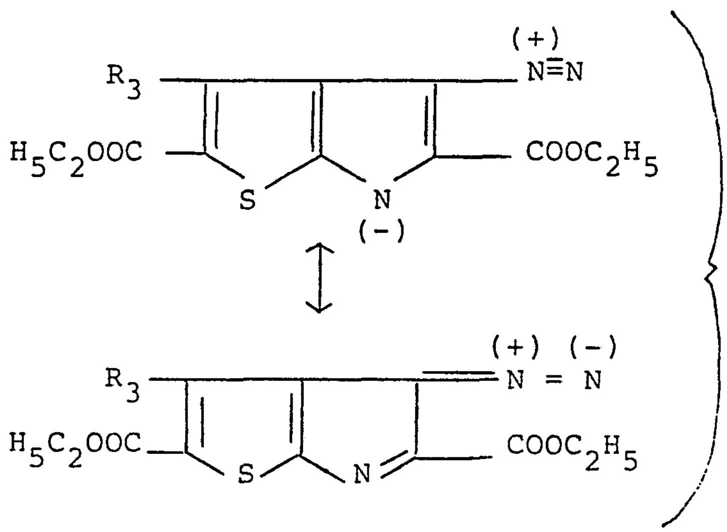

(II)

in which $R_3$ has the meaning defined above, are de-diazotised to obtain the compound of the general formula III:

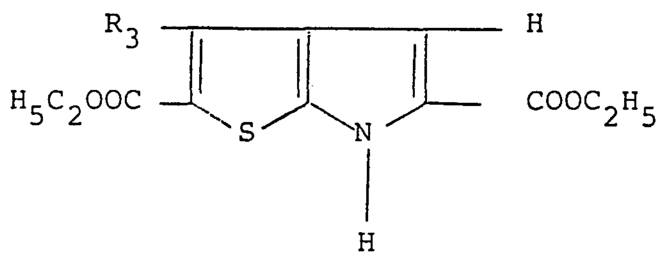

(III)

in which $R_3$ has the meaning given above,

the compound III so obtained is acylated according to the Friedel-Crafts method by means of an acyl halide of the formula $X\text{-}CO\text{-}R_4$ in which $R_4$ has the meaning defined above and X represents a halogen atom, to obtain the derivative of the general formula IV:

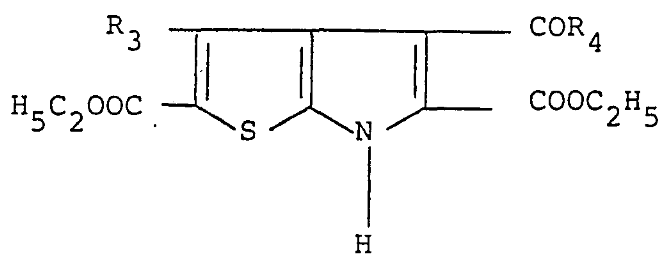

(IV)

in which $R_3$ and $R_4$ have the meanings given above,

the derivatives IV are hydrolysed to obtain the compounds of the general formula V:

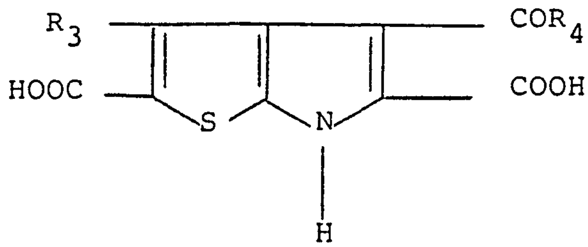

(V)

in which $R_3$ and $R_4$ have the meanings defined above, these compounds V are decarboxylated to obtain the derivatives of the general formula VI:

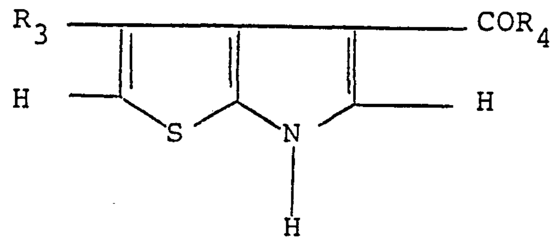

(VI)

in which $R_3$ and $R_4$ have the meanings defined above, which compounds VI are then N-alkylated by means of an α-halo acid or a derivative of the formula VII:

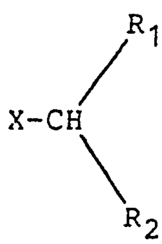

(VII)

in which $R_1$ and $R_2$ have the meanings defined above and X represents a halogen atom.

2. Process of preparation according to claim 1, characterised in that the de-diazotisation of the derivatives II is carried out by heating in an appropriate solvent, optionally with catalysis of the reaction by traces of HCl, U.V. or radical initiators, or by the action of a reducing agent.

3. Process according to claims 1 and 2, characterised in that the acylation of the compounds III is carried out in the presence of a catalyst in an appropriate solvent.

4. Process according to claims 1 and 3, characterised in that the hydrolysis of the compounds IV is carried out by heating the compounds IV in a base-ethanol mixture, followed by acidification.

5. Process according to claims 1 to 4, characterised in that the decarboxylation of the compounds V is carried out by heating in a suitable solvent in the presence of copper powder as catalyst.